# EUROPEAN PATENT SPECIFICATION

(11) **EP 2 863 976 B1**
(45) Date of publication and mention of the grant of the patent: **25.07.2018**
(21) Application number: 13854556.1
(22) Date of filing: 15.11.2013
(51) Int. Cl.: A61M 16/04, A61M 16/10

(54) **NASAL PULSATILE OXYGENATION AND VENTILATION AIRWAY APPARATUS**
PULSIERENDE NASALE SAUERSTOFFZUFUHR- UND BEATMUNGSVORRICHTUNG
APPAREIL D'OXYGÉNATION ET DE VENTILATION DE TYPE PULSATILE PAR VOIE NASALE DES VOIES AÉRIENNES NASALES

(30) Priority: 15.11.2012 US 201261726664 P; 13.03.2013 US 201361779379 P
(43) Date of publication of application: 29.04.2015
(73) Proprietor: The Trustees of The University of Pennsylvania, Philadelphia, PA 19104-6283 (US)
(72) Inventor: WEI, Huafeng, Cherry Hill, NJ 08003 (US)
(74) Representative: Pearl Cohen Zedek Latzer Baratz UK LLP
(86) International application number: PCT/US2013/070253
(87) International publication number: WO 2014/078631

(56) References cited:
- WO-A1-2008/144589
- WO-A1-2011/014543
- WO-A2-2005/107839
- US-A1- 2005 279 360
- US-A1- 2005 284 482
- US-A1- 2006 249 161
- US-A1- 2009 044 808
- US-A1- 2009 156 953
- US-A1- 2012 130 264

## Description

### FIELD OF THE INVENTION

The invention provides a nasal air jet insufflator (or jet nasal airway) apparatus for nasally delivering supraglottic jet oxygenation and ventilation and methods of use thereof.

### BACKGROUND OF THE INVENTION

Respiratory depression or "labored breathing" is frequently seen in patients or subjects with various kinds of diseases. Oxygen is usually provided to patients either through their nose or mouth to improve the oxygenation and correct hypoxia. However, in patients with severe respiration depression or apnea either under heavy sedation or light general anesthesia such as during colonoscopy, upper gastrointestinal endoscopy or cytoscopy or with some cardiopulmonary diseases, simple passive inhalation of oxygen via nose or mouth may not be adequate or impossible and thus result in hypoxia and/or hypercapnia, brain damage or even death. Tracheal intubation and mechanical ventilation can help patient's breath but this needs medical personnel with special training and the procedure is invasive and may have multiple complications related to tracheal intubation. Additionally, these patients usually need continuous heavy sedation, light general anesthesia or a muscle relaxant to keep and tolerate the endotracheal tube in the trachea after tracheal intubation.

Heavy sedations with intravenous infusion of general anesthetic propofol are frequently used to assist outpatient procedures (*e.g.,* endoscope examinations of gastrointestinal tracts or bile ducts, bronchoscope, endoscopy, cystoscopy, etc...). Propofol sedation can depress patient's breathing severely, especially when the patient is obese or in a prone position during endoscope examination or surgery in bile ducts. Passive inhalation of oxygen via nose or mouth using nasal cannula or nasal airway with patient's natural breathing is often not adequate to correct hypoxia and/or hypercapnia caused by heavy sedation. Mask ventilation is often impossible because of the endoscope in the mouth. Tracheal intubations and mechanical ventilation are usually not chosen because these procedures are often short and do not warrant full general anesthesia.

Hypoxia during tracheal intubation is the most common complications causing morbidity and mortality in the clinical practice of anesthesia, which is often related to an unexpected difficult airway. This can contribute to up to 28% of anesthesia related deaths. Occasionally, both mask ventilation and tracheal intubation are impossible in an apnea patient resulting in non-ventilation and non-intubation airway emergency. Lack of effective airway devices that maintain oxygenation and ventilation during the process of tracheal intubation in an apnea patient is still one of the primary causes of the morbidity and mortality during anesthesia practice. Accordingly, there exists a need for an improved airway apparatus capable of providing adequate oxygenation and ventilation before or during the process of tracheal intubation.

WO2011014543 (A1) discloses apparatus for nasally delivering a supraglottic jet ventilation and methods of treating breathing disorders by utilizing the apparatus for nasally delivering a supraglottic jet ventilation. WO2005107839 (A2) discloses an endotracheal device includes a unit with a pair of tubes and a beveled distal end. One tube is used for jet ventilation, while a monitoring catheter is provided inside the second, larger tube.

### SUMMARY OF THE INVENTION

The scope of the invention is as defined by the appended claims. Provided herein is an apparatus for nasally delivering a supraglottic jet ventilation, the apparatus comprising: (a) an elongated flexible tube having: (i) an annular cylindrical wall defining at least one tube lumen extending substantially the entire length thereof, said cylindrical wall having external and internal surfaces and having proximal and distal ends, (ii) a first catheter lumen extending lengthwise within said cylindrical wall between said external surface and said internal surface and along a dorsal region thereof, said first lumen having a first opening through the external surface of said cylindrical wall adjacent the proximal end thereof and a second opening through the internal surface of said cylindrical wall adjacent the distal end thereof, (iii) a second catheter lumen extending lengthwise within said cylindrical wall along a ventral region thereof, said lumen having a first opening through the external surface of said cylindrical wall adjacent the proximal end thereof and a second opening through a distal face of said cylindrical wall at the distal end of said cylindrical wall; (b) a first catheter extending dorsally through said first catheter lumen, said first catheter having a proximal end extending outside of said cylindrical wall through said first opening and having a distal end extending into said tube lumen through said second opening (c) a second catheter extending ventrally through said second catheter lumen, said second catheter having a proximal end extending outside of said cylindrical wall through said first opening and having a distal end extending through said second opening. The apparatus further comprises a breath sound sensor and/or pressure sensor located on the internal surface of said cylindrical wall; a marker present along the external surface of said cylindrical wall, wherein said marker comprises a ruler marking and is present on the proximal portion of said tube; and wherein the presence of said marker helps determine the depth of nasal airway in the nasal cavity; and a ring slidable along at least the proximal end of said elongated flexible tube or a movable stopper.

In some embodiments, the apparatus further comprises an inflatable balloon cuff extending substantially along the external surface of said cylindrical wall, wherein said balloon cuff is coupled to the distal end of said tube. The balloon cuff is capable of stopping nasal bleeding by inflating the cuff, thereby exerting pressure on nasal wall.

In other embodiments, the apparatus further comprises an attachment mechanism that is capable of securing the apparatus to a subject's face. The attachment mechanism may comprise a movable clip wrapped around the substantial portion of the external surface of said cylindrical wall. In an exemplary embodiment, a flexible wire or rope may be coupled to both ends of said movable clip in order to secure the apparatus to a subject's face after the placement of the apparatus on the subject's face.

In other embodiments, the apparatus further comprises a mechanism for synchronizing a subject's breath to a jet ventilator. In one example, the apparatus comprises a breath sound sensor located on the inner surface of said cylindrical wall. A breath sound detector may be coupled to said breath sound sensor, wherein said breath sound detector may also be coupled to a jet ventilator to initiate a jet pulse when the subject inhales or breathes in air. In another example, the capnogram generated from a PetCO₂ monitoring catheter imbedded in the apparatus can be used to trigger the jet ventilator and be synchronized to the subject's spontaneous breathing with the supraglottic oxygenation and ventilation. In yet another example, the apparatus comprises a pressure sensor located on the inner surface of said cylindrical wall to sense airway pressures. A pressure detector may be coupled to said pressure sensor, wherein said pressure detector may also be coupled to a jet ventilator to initiate a jet pulse when the subject inhales or breathes in air.

In some embodiments, the apparatus comprises the foregoing balloon cuff and further comprises at least one or more of the foregoing markers, the foregoing attachment mechanisms, and the foregoing mechanisms for synchronizing a subject's breath to a jet ventilator. In some embodiments, the apparatus comprises the foregoing marker and further comprises at least one or more of the foregoing balloon cuffs, the foregoing attachment mechanisms, and the foregoing mechanisms for synchronizing a subject's breath to a jet ventilator. In some embodiments, the apparatus comprises the foregoing attachment mechanism and further comprises at least one or more of the foregoing balloon cuffs, the foregoing markers, and the foregoing mechanisms for synchronizing a subject's breath to a jet ventilator. In some embodiments, the apparatus comprises the foregoing mechanism for synchronizing a subject's breath to a jet ventilator attachment mechanism and further comprises at least one or more of the foregoing balloon cuffs, the foregoing markers, and the foregoing attachment mechanisms.

In another aspect, provided herein are systems for ventilating and/or oxygenating a subject with compromised breathing, the systems comprising: (a) an apparatus described herein; (b) a jet ventilator or device; and (c) a CO₂ monitoring device. In another aspect, methods of synchronizing a subject's breathing with a jet ventilator are provided. In another aspect, provided herein are methods for treating a disease. Also provided are methods for making the apparatus.

Unless otherwise defined, all technical and scientific terms used herein have the same meaning as commonly understood by one of ordinary skill in the art to which this invention belongs. Other features and advantages of the present invention will become apparent from the following detailed description examples and figures. It should be understood, however, that the detailed description and the specific examples while indicating preferred embodiments of the invention are given by way of illustration only, since various changes and modifications within the scope of the invention will become apparent to those skilled in the art from this detailed description. It is also contemplated that whenever appropriate, any embodiment of the present invention can be combined with one or more other embodiments of the present invention, even though the embodiments are described under different aspects of the present invention.

### BRIEF DESCRIPTION OF THE DRAWINGS

The invention will be better understood from a reading of the following detailed description taken in conjunction with the drawings in which like reference designators are used to designate like elements, and in which:
Figure **1** is a schematic drawing of the apparatus, according to one embodiment of the invention.
Figure **2** is a schematic drawing of the apparatus, according to another embodiment of the invention.

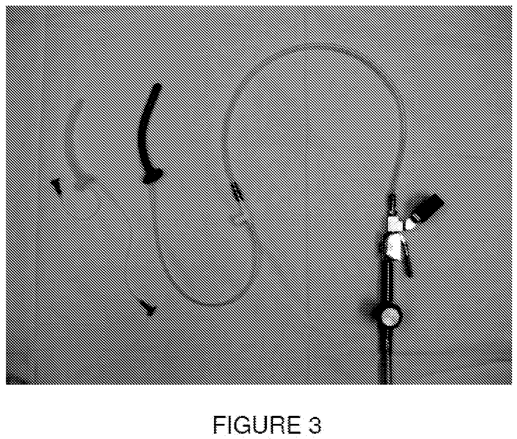

Figure **3** illustrates a jet nasal prototype using a regular nasal airway and a jet catheter inside its lumen.
Figure **4** illustrates a jet nasal airway device, according to one embodiment of the invention.
Figure **5** illustrates a closer view of the jet nasal airway device of Figure **4****.**

### DETAILED DESCRIPTION OF THE INVENTION

The invention provides an apparatus for nasally delivering a supraglottic jet oxygenation and ventilation and methods of use thereof.

The apparatus includes an elongated flexible tube having: (i) an annular cylindrical wall defining at least one tube lumen extending substantially the entire length thereof, said cylindrical wall having external and internal surfaces and having proximal and distal ends, (ii) a first catheter lumen extending lengthwise within said cylindrical wall between said external surface and said internal surface and along a dorsal region thereof, said first lumen having a first opening through the external surface of said cylindrical wall adjacent the proximal end thereof and a second opening through the internal surface of said cylindrical wall adjacent the distal end thereof, (iii) a second catheter lumen extending lengthwise within said cylindrical wall along a ventral region thereof, said lumen having a first opening through the external surface of said cylindrical wall adjacent the proximal end thereof and a second opening through a distal face of said cylindrical wall at the distal end of said cylindrical wall; (b) a first catheter extending dorsally through said first catheter lumen, said first catheter having a proximal end extending outside of said cylindrical wall through said first opening and having a distal end extending into said tube lumen through said second opening (c) a second catheter extending ventrally through said second catheter lumen, said second catheter having a proximal end extending outside of said cylindrical wall through said first opening and having a distal end extending through said second opening

In one aspect, the apparatus comprises an inflatable balloon cuff that extends substantially along the external surface of the cylindrical wall. The inflatable balloon cuff may be coupled to the distal end of the tube. The inflatable balloon cuff is capable of stopping nasal bleeding by exerting pressure on the nasal wall inside the nasal cavity.

In another aspect, the apparatus comprises a marker present along the external surface of the cylindrical wall. The marker comprises a ruler marking, for example, in centimeters (cm), millimeters (mm), or a combination thereof. The marker may be present on the proximal portion of the tube. The presence of the marker helps determine the depth of nasal airway in the nasal cavity. Preferably, the distal end of the jet nasal airway should pass the posterior nostril so that high speed flow jet pulses will not be applied to the mucus membrane on the inner wall of nasal cavity and cause possible nasal bleeding. Therefore the apparatus should preferably be long enough to reach above the vocal cord so that the length of the apparatus in the nasal cavity can be adjusted to make the distal end of jet nasal airway apparatus pointing directly or close to the vocal cord opening to optimize the effects of active supraglottic jet oxygenation and ventilation. The inner diameter (ID) should preferably be small enough so that it will facilitate the insertion of the apparatus while minimizing the chance of bleeding during placement of the apparatus in the nasal cavity.

In yet another aspect, the apparatus comprises an attachment mechanism that is capable of securing the apparatus to a subject's face. The attachment mechanism includes a movable clip wrapped around a substantial portion of the external surface of the cylindrical wall. A flexible wire or rope may be coupled to both ends of said movable clip in order to secure the apparatus to the subject's face after the placement of the apparatus on the subject's face.

In addition, according to another aspect of the invention, the apparatus includes a breath sound or pressure detection mechanism to detect inhaling and/or exhaling breath sound. A breath sound or pressure sensor may be located on the inner surface of the tube lumen. The sound or pressure detector can be directly or indirectly coupled, respectively, to the breath sound or pressure sensor, for example, through an electric wire, and also can be directly or indirectly coupled to a jet ventilator to initiate a jet pulse so that the jet ventilator will provide an active jet pulse into trachea via supraglottic jet oxygenation and ventilation when the subject inhales or breaths in.

In some embodiments, the apparatus of the invention may include a plurality of catheter lumens, each extending lengthwise within the cylindrical wall between the external surface and the internal surface. In one example, the apparatus of the invention may include a first catheter lumen and a second catheter lumen. The first catheter lumen may extend lengthwise along a dorsal region of the tube and the second catheter lumen may extend lengthwise along a ventral region of the tube.

The first catheter lumen comprises a first opening through the external surface of the cylindrical wall adjacent to the proximal end thereof and a second opening through the internal surface of the cylindrical wall adjacent to the distal end thereof. The second catheter lumen also comprises a first opening through the external surface of the cylindrical wall adjacent to the proximal end thereof and a second opening through a distal face of the cylindrical wall at the distal end of the cylindrical wall.

The first catheter lumen may include a first catheter that may extend dorsally through said first catheter lumen. The first catheter has a proximal end extending outside of the cylindrical wall through the first opening and a distal end extending into the tube lumen through the second opening.

The second catheter lumen may include a second catheter that may extend ventrally through said second catheter lumen. The second catheter has a proximal end extending outside of the cylindrical wall through the first opening and a distal end extending through the second opening.

In certain embodiments, the first catheter is adapted to accommodate a jet ventilator, a jet device or oxygen insufflation. In some embodiments, the first catheter is adapted to accommodate a CO₂ monitor or sensor. The CO₂ signal can also be used to trigger the jet ventilator so that the jet pulses are injected when the subject inhales or breaths in and supraglottic jet oxygenation and ventilation is synchronized with the subject's spontaneous breathing.

In certain embodiments, the second catheter is adapted to monitor CO₂. In some embodiments, the second catheter is adapted to accommodate a jet ventilator. In some embodiments, the second catheter is adapted to accommodate a jet device. In some embodiments, the second catheter is adapted to accommodate oxygen insufflation.

In some embodiments, the jet catheter extends beyond the distal end of the tube. In one example, the jet catheter may extend such that the distal end of the jet catheter is flush with the distal end of the tube. In some embodiments, the distal end of the jet catheter is set back relative to the distal end of the catheter adapted to monitor CO₂ in the direction of the tube lumen proximal end. For example, the jet catheter may be set back in order that the jet pulses are not directed below the vocal cord, thereby minimizing the amount of the jet pulse directed into the esophagus.

The inner diameter of the first and second catheter lumens (*e.g.,* the jet catheter lumen or the CO₂ monitoring catheter lumen) may be smaller than the inner diameter of the tube lumen. In certain embodiments, the first catheter lumen or the second monitoring catheter lumen has an inner diameter of between about 0.1 mm and 2.5 mm and the tube lumen has an inner diameter of between about 1 mm and 10 mm.

In some embodiments, the apparatus further comprises a visual monitoring unit for observing the vocal cord. In certain embodiments, the visual monitoring unit is positioned within the tube lumen.

In some embodiments, the apparatus further comprises a jet ventilation source for providing jet ventilation through the jet catheter and/or CO₂ monitoring catheter. The jet ventilation source may be controlled for jet pulse frequency, pulse pressure, inspiratory/expiratory ratio (I/E) ratio, and the oxygen concentrations in the jet pulse.

The length of the apparatus may range between about 2 and 20 cm. The apparatus is capable of being used on subjects of many age groups, including adult, child, and infant subjects.

Provided herein are methods of oxygenating and/or ventilating a subject, where the subject is afflicted with a disease or condition, is undergoing tracheal intubation, or is under heavy sedation during a surgery, which results in compromised breathing. The methods includes the step of positioning or placing a nasal jet airway device described herein, using an attachment mechanism described herein, on the subject for nasally delivering supraglottic jet pulses of air, oxygen, or mixtures thereof at various concentrations. Also, provided herein are methods of synchronizing the jet pulses to the foregoing subjects

The methods may include adjusting the flexible wire or rope to adjust the movable clip in order to secure the apparatus to a subject's face so that it will not move around after the distal end of jet nasal airway apparatus has been properly placed in the nasal cavity.

In some aspects, when there is a nasal bleeding, the methods include the step of inflating the balloon cuff to exert pressure on the nasal wall, and thereby stop the bleeding.

In some aspects, the method includes the step of measuring the depth of nasal airway in the nasal cavity using the ruler markings of the marker. Initial depth of the nasal airway with active oxygenation and ventilation in the nasal cavity can be the distance between ear and nostril on the same side. The depth of the nasal airway with active oxygenation and ventilation in the nasal cavity can be adjusted by moving in or out of the nasal cavity to obtain, for example, the maximum levels of chest rise, breath sound, or end-tidal CO₂.

In some aspects, the methods include the step of detecting the inhaling and exhaling breath sound or the end tidal CO₂ of a subject and synchronizing the subject's spontaneous breathing with a jet ventilator.

In some aspects, the methods further comprise the step of adjusting the position of the jet pulse in the nasal airway to optimize the end-tidal CO₂ with a capnogram having a stable plateau. In some aspects, the methods further comprise the step of observing the subject's vocal cord by at least one visual monitoring unit, such as a fiber-optic scope.

In some aspects, the methods further comprise the step of changing jet ventilation through the jet catheter to the CO₂ monitoring catheter, and using jet catheter for CO₂ monitoring. In some aspects, the choice of the catheter function depends on best chest rise and maximum end-tidal CO₂ obtained with use of each of two catheters.

In some aspects, the methods further comprise the step of capping the proximal end of the jet catheter. In some aspects, the method further comprises the step of capping the proximal end of the CO₂ monitoring catheter. In some aspects, methods further comprise the step of providing conventional ventilation using a breathing bag to the subject through the first tube. In some aspects, the methods further comprise the step of delivering medication to the subject through the jet catheter. In some embodiments, the methods further comprise the step of delivering medication to the subject through the CO₂ monitoring catheter. In some aspects, the methods further comprise the step of applying suction forces to the nasal airway in a subject through the jet catheter or CO₂ monitoring catheter.

The apparatus of the invention can be used to treat any suitable disease or condition. For example, without limitation, the disease or condition is respiratory depression, apnea, hypoxia, hypercapnia, or any combination thereof.

Provided are systems for ventilating a subject with compromised breathing. The subject with compromised breathing may, for example, be a subject afflicted with a disease or condition, or is a subject undergoing tracheal intubation, or is a subject under heavy sedation during a surgery. The system may include the jet nasal airway device described herein.

In certain embodiments, the systems further comprise a unit for viewing a vocal cord, such as a fiber optic unit.

In some embodiments, the systems further comprise a jet ventilator. The jet ventilator can be any device that generates jet pulses. In some embodiments, the systems further comprise a CO₂ monitoring unit. The CO₂ monitoring unit is capable of monitoring end-tidal CO₂.

In some embodiments, the system further comprises a mechanism for applying suction to the apparatus through the tube lumen. In certain embodiments, the system further comprises a central control unit. In some embodiments, the central control unit comprises a sensor of breathing, a computer to integrate breathing signal and provide triggering signal for jet ventilator to synchronize the jet pulse from the jet ventilator with spontaneous breathing of subject.

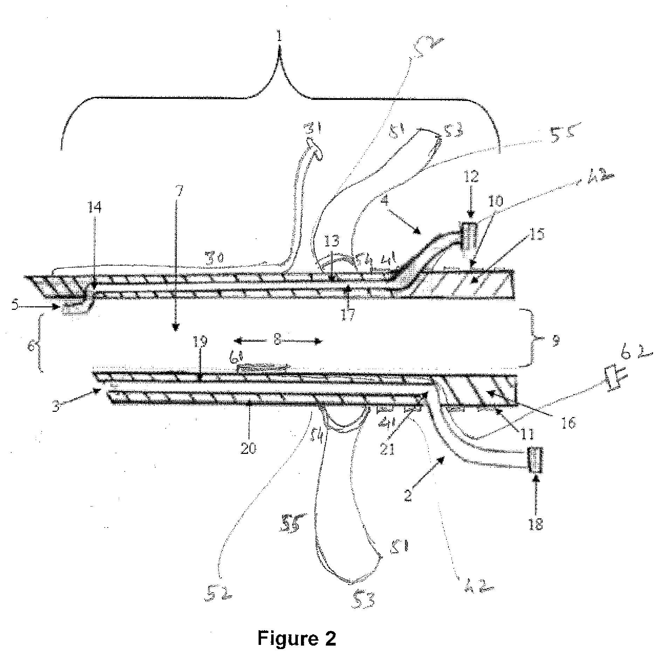

In certain embodiments, as shown in Figure 2, provided herein is a jet nasal airway device comprising a nasal airway unit (1) comprising: a tube (7) having a proximal end (9), a distal end (6), an anterior surface (10), and a posterior surface (11), a first tube wall (15) and a second tube wall (16) enclosing a tube lumen (8); a jet catheter (4) partially enclosed within the first tube wall (15) having proximal end (12), a distal end (5) and comprising a first jet catheter wall and a second jet catheter wall (13 and 17, respectively) enclosing a jet catheter lumen (14), wherein the proximal end (12) of jet catheter extends outwards from the first tube wall (15), wherein the distal end (5) of jet catheter extends inwards from the first tube wall (15) into the tube lumen; an end-tidal CO₂ monitoring catheter (2), partially enclosed within the second tube wall (16) having proximal end (18), a distal end (3) and comprising a first end-tidal CO₂ monitoring catheter wall and a second end-tidal CO₂ monitoring catheter wall (19, 20) enclosing an end-tidal CO₂ monitoring catheter lumen (21), wherein the proximal end (18) of an end-tidal CO₂ monitoring catheter extends outwards from the second tube wall (16), wherein the distal end (3) of an end-tidal CO₂ monitoring catheter is located within the second tube wall (16).

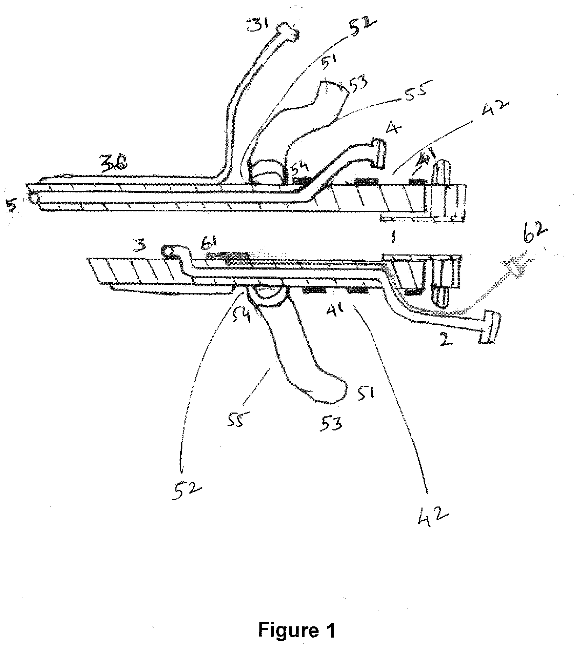

As shown in the embodiments of Figures 1 and 2, the nasal airway device also comprises an inflatable balloon cuff (30), a marker (41), an attachment mechanism (51), a breath sound sensor (61) and a breath sound detector (62). The balloon cuff (31) may be coupled to the distal end (6) of the tube (7).

The marker (41) includes a ruler marking (42), for example, in centimeters, millimeters, or other distance measuring markings. The marker (41) may be present in the proximal portion of the tube (9).

The attachment mechanism (51) includes a movable clip (52) wrapped around a substantial portion of the external surface of the cylindrical wall (15). Both ends (53, 54) of movable clip (52) may be coupled to a flexible wire or rope (55) so that the device can be secured to the subject's face.

A breath sound sensor (61) may be located on the inner surface of the tube lumen (8). A sound detector (62) can be directly or indirectly coupled to the breath sound sensor (61), for example, through an electric wire, and also can be directly or indirectly coupled to a jet ventilator to initiate jet pulse so that the jet ventilator will provide active jet pulse into trachea via supraglottic jet ventilation when the subject inhales or breaths in. Alternatively or additionally, a pressure sensor (not shown) may be located on the inner surface of the tube lumen (7). A pressure detector (not shown) can be directly or indirectly coupled to the breath sound sensor, for example, through an electric wire, and also can be directly or indirectly coupled to a jet ventilator to initiate jet pulse so that the jet ventilator will provide active jet pulse into trachea via supraglottic jet ventilation when the subject inhales or breaths in. Accordingly, jet ventilation is synchronized with the subject's breathing so that jet ventilator may inject active jet pulses when the subject is inhaling or breathing in.

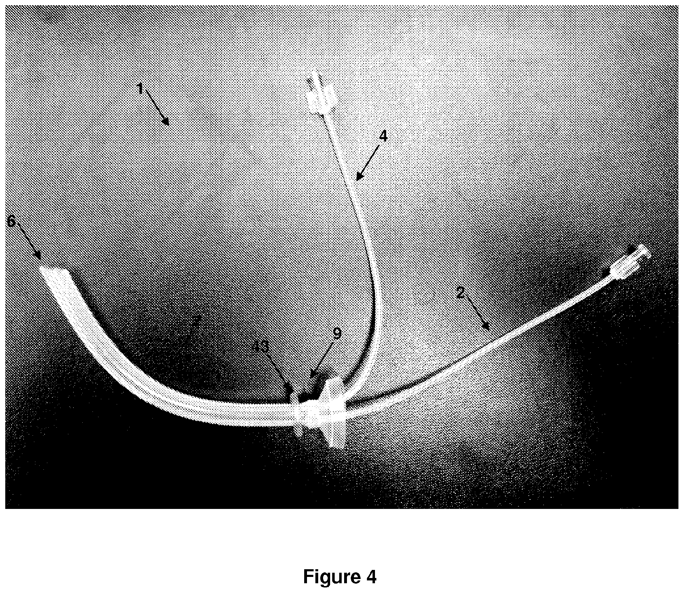

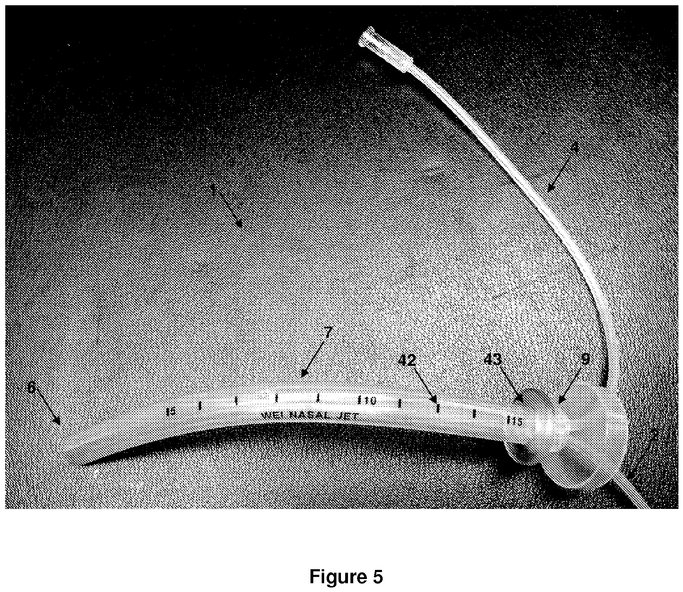

Figures 4 and 5 depict a jet nasal airway device (1) according to certain embodiments. Jet nasal airway device (1) comprises: a tube (7) having a proximal end (9) and a distal end (6); a jet catheter (4); and an end-tidal CO₂ monitoring catheter (2). The device (1) also includes ruler markings (42), for example, in centimeters or other distance measuring markings, on a surface of tube (7). The nasal airway device (1) also comprises a stopper or ring (43), such as a plastic ring, surrounding the tube (7) near its proximal end (9). Ring (43) can slide axially along at least the proximal end (9) of tube (7) to loosely maintain the depth of device in the nasal cavity after proper airway placement. In use, prior to insertion of the device into a subject, the ring may be positioned or located at a position near the proximal end of the tube. The device may then be inserted into the nasal cavity of a subject to a depth greater, *e.g.,* 2 to 4 cm, than ultimately desired. The device is then extruded while adjusting its position (*e.g.,* by rotation) to achieve, for example, the maximum levels of chest rise, breath sound, or end-tidal CO₂. Once the device is properly positioned, the ring may be slid distally until it loosely abuts the nostril to maintain the device at the desired depth within the nasal cavity.

In some embodiments, the proximal end of the end-tidal CO₂ monitoring catheter is capped. In some embodiments, the proximal end of the end-tidal CO₂ monitoring catheter is cuffed. In some embodiments, the proximal end of the jet catheter is capped. In some embodiments, the proximal end of the jet catheter is cuffed. The end-tidal CO₂ monitoring can be can be directly or indirectly coupled to a jet ventilator to initiate jet pulse so that the jet ventilator will provide active jet pulse into trachea via supraglottic jet ventilation when the subject inhales or breaths in. Accordingly, in certain embodiments, jet ventilation is synchronized with the subject's breathing so that jet ventilator may inject active jet pulses when the subject is inhaling or breathing in.

As shown in the embodiment of Figure 1, the nasal airway unit (1) comprises inner lumen at the proximal end with active oxygenation and ventilation (away from the subject's vocal cord) features and an adaptor for connecting to a conventional mechanical ventilator to perform conventional mechanical ventilation such as pressure- or volume- controlled ventilation if needed. The apparatus also comprises proximal end of the built-in jet catheter (4) or end-tidal CO₂ monitoring catheter, which can be coupled to a jet ventilator and/or end tidal CO₂ monitor; distal end (5) of the built-in jet or end-tidal CO₂ monitoring catheter; proximal end of the built-in end-tidal CO₂ monitoring or jet catheter (2), which can be coupled to an end tidal CO₂ monitor and/or a jet ventilator; distal end (5) of the built-in end-tidal CO₂ monitoring or jet catheter; a balloon cuff (30) on the outer surface of the nasal airway with active oxygenation and ventilation to treat occasional nose bleeding or assist conventional mechanical ventilation; proximal end (31) of the tube connecting to balloon cuff for inflation of balloon cuff if needed; marker (41) in numbers, for example, in centimeters to indicate distance between marker and the most distal end (5) of the nasal airway with active oxygenation and ventilation; movable stopper or a clip (55) wrapping around outer surface of the nasal airway with active oxygenation and ventilation with tape or rope on both end to secure the device on the subject's face after proper airway placement; and breath sound sensor (61) located on the inner surface of the lumen in the nasal airway with active oxygenation and ventilation to detect the subject's inhaling and exhaling breath sound. The distal end (62) of the breath sound detector can be coupled to the breath sound sensor through an electric wire and also can be coupled to a jet ventilator to initiate jet pulse so that jet ventilator will provide active jet pulse into the trachea via supraglottic jet ventilation when the subject inhales or breaths in.

The terms "apparatus for nasally delivering a jet of air or oxygen" or "jet nasal airway device" or "supraglottic jet nasal airway" or "nasal jet airway device" are used interchangeably. Various features of a nasal jet airway device are described in PCT patent application publication WO 2011/014543 and U.S. patent application publication U.S. 2012/0130264.

In certain embodiments, the first catheter is adapted to accommodate an air or oxygen jet. In some embodiments, the second catheter is adapted to monitor CO₂. In some embodiments, the second catheter is an end-tidal CO₂ monitoring catheter. In some embodiments, the jet nasal airway device further comprises a jet ventilation source for providing jet oxygenation and ventilation through the jet catheter. In one aspect, the jet nasal airway device further comprises an inflatable insufflation cuff.

In some embodiments, the inner diameter of the jet catheter lumen is smaller than the inner diameter of the tube lumen. In some embodiments, the first tube wall (15) has a protruding end. In some embodiments, the second tube wall (16) has a protruding end. In some embodiments, the distal end of the tube lumen is beveled. For example, the distal end of the tube lumen is beveled for ease of insertion of the apparatus into the nasal airway of the subject. In some embodiments, the distal end of the tube lumen is beveled such that the dorsal end is longer than the ventral end. In some embodiments, the distal end of the tube lumen is beveled such that the ventral end is longer than the dorsal end. In some embodiments, the distal end of the tube lumen is beveled at angle to the dorsal-ventral axis.

In some embodiments, the jet catheter lumen (14) has an inner diameter of 0.1 mm to 2.3 mm. In some embodiments, the jet catheter lumen has an inner diameter of 0.5 mm to 1.5 mm. In some embodiments, the jet catheter lumen has an inner diameter of 0.1 mm to 2.5 mm. In some embodiments, the jet catheter lumen has an inner diameter of 1 mm to 1.5 mm. In some embodiments, the jet catheter lumen has an inner diameter of 1.5 mm to 2.3 mm. In some embodiments, the jet catheter lumen has an inner diameter of 1.5 mm to 2.5 mm.

In some embodiments, the end-tidal CO₂ monitoring catheter (21) has an inner diameter of 0.1 mm to 4 mm. In some embodiments, the end-tidal CO₂ monitoring catheter has an inner diameter of 0.5 mm to 1.5 mm. In some embodiments, the end-tidal CO₂ monitoring catheter has an inner diameter of 0.1 mm to 2.5 mm. In some embodiments, the end-tidal CO₂ monitoring catheter has an inner diameter of 1 mm to 3 mm. In some embodiments, the end-tidal CO₂ monitoring catheter has an inner diameter of 2.5 mm to 3.5 mm. In some embodiments, the end-tidal CO₂ monitoring catheter has an inner diameter of 2.5 mm to 4 mm.

In some embodiments, the tube's lumen has an inner diameter of 1 mm to 12 mm. In some embodiments, the tube's lumen has an inner diameter of 1 mm to 9 mm. In some embodiments, the tube's lumen has an inner diameter of 1 mm to 3 mm. In some embodiments, the tube's lumen has an inner diameter of 2 mm to 4 mm. In some embodiments, the tube's lumen has an inner diameter of 3 mm to 5 mm. In some embodiments, the tube's lumen has an inner diameter of 4 mm to 6 mm. In some embodiments, the tube's lumen has an inner diameter of 5 mm to 7 mm. In some embodiments, the tube's lumen has an inner diameter of 6 mm to 8 mm. In some embodiments, the tube's lumen has an inner diameter of 7 mm to 9 mm. In some embodiments, the tube's lumen has an inner diameter of 3 mm to 8 mm. In some embodiments, the tube's lumen has an inner diameter of 2 mm to 5 mm. In some embodiments, the tube's lumen has an inner diameter of 6 mm to 9 mm.

In some embodiments, the jet nasal airway device comprises an inflatable balloon cuff that extends substantially along the external surface of the cylindrical wall. The inflatable balloon cuff may be coupled to the distal end of the tube. The inflatable balloon cuff is capable of stopping nasal bleeding by exerting pressure on the nasal wall inside the nasal cavity. In use, the length of the ballon cuff may be almost the entire depth of the device inserted into the nasal cavity. In some cases, the balloon cuff length is at least 95% of the nasal airway depth inserted by the device. In some cases, the balloon cuff length is at least 90% of the nasal airway depth inserted by the device. In some cases, the balloon cuff length is at least 85% of the nasal airway depth inserted by the device. In some cases, the balloon cuff length is at least 80% of the nasal airway depth inserted by the device. In some cases, the balloon cuff length is at least 75% of the nasal airway depth inserted by the device. In some cases, the balloon cuff length is at least 70% of the nasal airway depth inserted by the device. In some cases, the balloon cuff length is at least 65% of the nasal airway depth inserted by the device. In some cases, the balloon cuff length is at least 60% of the nasal airway depth inserted by the device. In some cases, the balloon cuff length is at least 55% of the nasal airway depth inserted by the device. In some cases, the balloon cuff length is at least 50% of the nasal airway depth inserted by the device.

In some embodiments, the jet nasal airway device further comprises a visual monitoring unit for observing the vocal cord, such as an optic fiber.

In some embodiments, the visual monitoring unit is attached to the jet nasal airway device. In some embodiments, the visual monitoring unit is included within the jet nasal airway device. In some embodiments, the visual monitoring unit is positioned within the tube's lumen.

In some embodiments, the jet nasal airway device is compressible. In some embodiments, the jet nasal airway device is flexible. In some embodiments, the jet nasal airway device is elastic.

In some embodiments, the jet nasal airway device has a length of 2-20 cm. In some embodiments, the jet nasal airway device has a length of 2-5 cm. In some embodiments, the jet nasal airway device has a length of 4-8 cm. In some embodiments, the jet nasal airway device has a length of 5-10 cm. In some embodiments, the jet nasal airway device has a length of 8-12 cm. In some embodiments, the jet nasal airway device has a length of 10-15 cm. In some embodiments, the jet nasal airway device has a length of 12-20 cm.

In some embodiments, the device is an implantable device that is positioned within an anatomical cavity of the nose.

In some embodiments, the device is comprised of a biocompatible material. In some embodiments, the device is comprised of a combination of biocompatible materials. In some embodiments, the device is comprised of a biocompatible material that provides the necessary physical properties for the device of the invention. In some embodiments, the device is comprised of a polymeric material (both natural and synthetic), a polymeric fiber, a ceramic material, a composite material, a metal, a metal oxide, and combinations thereof. In some embodiments, the device is comprised of amylose and amylopectin derivatives, polyamides, polyvinyl alcohol, polyvinyl acetals, polyvinylpyrrolidone, polyacrylates, epoxy resins, and polyurethanes (mixtures thereof, blends with other ingredients, or copolymers thereof) and combinations thereof.

In some embodiments, the device is coated. In some embodiments, the device is coated with a polymer or coating composition. In some embodiments, the device is coated with hyaluronic acid. In some embodiments, the device is coated with Perylenem™. In some embodiments, the device is coated with heparin. In some embodiments, the device is coated with a lubricant. In some embodiments, the device is coated with a thrombo-prevention compound. In some embodiments, the device is coated with an anti-bacterial compound. In some embodiments, the device is coated with a vaso-constriction medicine, such as neosynephrine, to prevent nose bleeding. In some embodiments, the device is coated with an anti-inflammatory compound. In some embodiments, the device is cross-linked or bound to a drug by gamma irradiation, chemical binding (as with binder or crosslinking molecules such as N-hydroxysuccinimide), or any other method. In some embodiments, the device is capable of the controlled release of a drug such as a surfactant, lubricant, antibiotic, anti-acid, antifungal agent, anti-inflammatory, or the like.

In some embodiments, the device is formed in part or in whole from a number of materials. In some embodiments, the materials are typically selected so as to ensure optimal device performance given the particular construction and/or geometry of the device. In some embodiments, the materials are tailored to the environment conditions to which the device may be exposed. In some embodiments, the environmental conditions of the nose may vary according to a number of factors, e.g., the particular temperature of the animal whose nose is to receive the device, whether the animal is healthy or diseased, whether pus or other bodily fluids are present, edema of the mucosa, etc.

In some embodiments, the device is substantially uniform in composition. In some embodiments, the device comprises of a plurality of regions that form an integrated whole. In some embodiments, the device is comprised of an interior region and a peripheral region, wherein the regions exhibit different compositions. In some embodiments, the peripheral region is formed from a biocompatible material. In some embodiments, the microstructure of the materials used with the invention is controlled in order to produce a device of controlled mechanical properties (e.g., tensile strength, elasticity). In some embodiments, the material is typically synthetic or man-made. In some embodiments, naturally occurring composition are used. In some embodiments, biocompatibility requires a material purity of a pharmaceutically acceptable grade.

In some embodiments, the material is a hydrophilic polymer. In some embodiments, the material hydrophilic polymers include polyethylene glycol, polyoxyethylene, polymethylene glycol, polytrimethylene glycols, polycinylpyrrolidones, and derivatives thereof. In some embodiments, the polymers are linear or multiply branched. In some embodiments, the material is polyethylene glycol (PEG) containing compound. In some embodiments, the material is a polyvinyl alcohol, polyacrylic acid, polyglycolic acid, polydioxanone. In some embodiments, the material is a biodegradable material such as polyesters of an α-hydroxy acids, lactic acid, glycolic acid, lactic esters, caprolactone, polyether-polyester combinations especially of polyethylene glycol (PEG) and aliphatic polyesters like poly (lactic acid), poly (glycolic acid) and poly (caprolactone), either as a blend or as a copolymer, in order to increase the hydrophilicity and degradation rate. In some embodiments, the material is a biodegradable polyanhydrides or polyorthoesters having labile backbone linkages.

In some embodiments, the material is a polysaccharide. In some embodiments, the material is hyaluronic acid. In some embodiments, the material is cyclodextrin. In some embodiments, the material is hydroxymethylcellulose. In some embodiments, the material is cellulose ether. In some embodiments, the material is a glycan. In some embodiments, the material is a collagen and other collagenic (collagen-like) materials

In some embodiments, the device is used in conjunction with pharmaceutical technologies known in the art. In some embodiments, a pharmacologically active constituent is bound to the device member or may be eludable. In some embodiments, such pharmacologically active constituents may promote healing and may include, for example, antibiotics, antifungal agent, anti-inflammatory, or the like. In some embodiments, the biocompatible material may be free from any pharmacologically active constituents.

In some embodiments, the device comprises a pharmaceutical substance that treats or prevents a microbial infection, the substance delivered may comprise pharmaceutically acceptable salt or dosage form of an antimicrobial agent (e.g., antibiotic, antiviral, antiparacytic, antifungal, etc.), a corticosteroid or other anti-inflammatory (e.g., an NSAID), a decongestant (e.g., vasoconstrictor), a mucous thinning agent (e.g., an expectorant or mucolytic), an agent that prevents of modifies an allergic response (e.g., an antihistamine, cytokine inhibitor, leucotriene inhibitor, IgE inhibitor, immunomodulator), etc.

In some embodiments, the device is inserted for a long period of time. In some embodiments, the device remains in the nose for a long period of time. In some embodiments, the device remains in the nose for at least one year. In some embodiments, the device remains in the nose for at least two years. In some embodiments, the device remains in the nose for at least three years.

In some embodiments, the device remains in the nose for at least a month. In some embodiments, the device remains in the nose for at least three months. In some embodiments, the device remains in the nose for at least four months. In some embodiments, the device remains in the nose for at least five months. In some embodiments, the device remains in the nose for at least seven months.

In some embodiments, the device remains in the nose for at least an hour. In some embodiments, the device remains in the nose for at least a day. In some embodiments, the device remains in the nose for at least three days. In some embodiments, the device remains in the nose for at least four days. In some embodiments, the device remains in the nose for at least a week. In some embodiments, the device remains in the nose for at least two weeks.

In some embodiments, the device is degraded at a programmed rate. In some embodiments, the device is designed to degrade at a rate wherein structure may be completely removed by aqueous solution flushing. In some embodiments, the device maintains sufficient structural integrity to maintain potency for a designed period of time. In some embodiments, the period of treatment may be for a period between two weeks, two months, six months, twelve months or more.

In some embodiments, a measure of the ability to maintain structural integrity would be that the device can sustain a radially applied force without breaking (after the defined period of time) that is at least one-half of the structural force that can be sustained prior to implantation or immersion in a test environment.

In some embodiments, it is well-known in the art that chemical materials, including lubricants, medicaments, and the like, may be dissolved or dispersed in a polymer and this will bloom or exude or migrate from the polymer for local delivery of the material.

In some embodiments, the device provides high frequency jet ventilation (HFJV) or low frequency jet ventilation (LFJV) characterized by its opening system, low tidal volume and low airway pressure. In some embodiments, the device maintains effective oxygenation and/or ventilation.

Having described preferred embodiments of the invention with reference to the accompanying drawings, it is to be understood that the invention is not limited to the precise embodiments, and that various changes and modifications may be effected therein by those skilled in the art without departing from the scope of the invention as defined in the appended claims.

## Claims

1. An apparatus for nasally delivering a supraglottic jet ventilation, the apparatus comprising:
a. an elongated flexible tube (7) having:
i. an annular cylindrical wall defining at least one tube lumen extending substantially the entire length thereof, said cylindrical wall having external (10) and internal (11) surfaces and having proximal (9) and distal (6) ends,
ii. a first catheter lumen (14) extending lengthwise within said cylindrical wall between said external surface and said internal surface and along a dorsal region thereof, said first lumen having a first opening through the external surface of said cylindrical wall adjacent the proximal end thereof and a second opening through the internal surface of said cylindrical wall adjacent the distal end thereof,
iii. a second catheter lumen (21) extending lengthwise within said cylindrical wall along a ventral region thereof, said lumen having a first opening through the external surface of said cylindrical wall adjacent the proximal end thereof and a second opening through a distal face of said cylindrical wall at the distal end of said cylindrical wall;
b. a first catheter (4) extending dorsally through said first catheter lumen, said first catheter having a proximal end extending outside of said cylindrical wall through said first opening and having a distal end extending into said tube lumen through said second opening;
c. a second catheter (2) extending ventrally through said second catheter lumen, said second catheter having a proximal end extending outside of said cylindrical wall through said first opening and having a distal end extending through said second opening; and
d.
i. a breath sound sensor (61) and/or pressure sensor located on the internal surface of said cylindrical wall;
ii. a marker (41) present along the external surface of said cylindrical wall, wherein said marker comprises a ruler marking and is present on the proximal portion of said tube; and wherein the presence of said marker helps determine the depth of nasal airway in the nasal cavity; and
iii. a ring (43) slidable along at least the proximal end of said elongated flexible tube or a movable stopper.

2. The apparatus of claim 1, wherein the apparatus further comprises a breath sound detector (62) coupled to said breath sound sensor (61) and/or a pressure detector coupled to said pressure sensor, respectively, and wherein said detector is coupled to a jet ventilator to initiate a jet pulse when a subject inhales or breathes in air.

3. The apparatus of any of claim 1 or claim 2, wherein the apparatus further comprises a balloon cuff (31), and wherein said balloon cuff, upon inflation, is suitable for stopping nasal bleeding by exerting pressure on nasal wall.

4. The apparatus of any of claims 1-3, wherein the apparatus further comprises an attachment mechanism (51) and wherein said attachment mechanism comprises a movable clip (52) wrapped around a portion of the external surface of said cylindrical wall, and further comprises a flexible wire or rope coupled to both ends of said movable clip suitable to secure the apparatus to the subject's face after the placement of the apparatus on the subject's face.

5. The apparatus of any of claims 1-4, wherein the first catheter is adapted to accommodate a jet ventilator or oxygen insufflator and the second catheter is adapted to monitor CO₂, or wherein the first catheter is adapted to monitor CO₂ and the second catheter is adapted to accommodate a jet ventilator or oxygen insufflator.

6. The apparatus of claim 5, wherein the distal end of the one of the first and second catheters, which is adapted to accommodate the jet ventilator or oxygen insufflator, is set back relative to the distal end of the other one of the first and second catheters, which is adapted to monitor CO₂, in the direction of the tube lumen proximal end.

7. The apparatus of any of claims 1-6, further comprising a visual monitoring unit for observing the vocal cords.

8. The apparatus of claim 7, wherein said visual monitoring unit is positioned within said tube lumen.

9. The apparatus of claim 5, further comprising a jet ventilation source for providing jet ventilation through the one of the first and second catheters adapted to accommodate a jet ventilator or oxygen insufflator, wherein said CO₂ monitor is coupled to the jet ventilation source to initiate a jet pulse when a subject inhales or breathes in air, and wherein the jet ventilation source is optionally controlled for jet pulse frequency, pulse pressure, inspiratory/expiratory ratio (I/E) ratio, and the oxygen concentrations in the jet pulse.

10. A system for ventilating and/or oxygenating a subject with compromised breathing, the system comprising:
a. the apparatus of claim 5;
b. a jet ventilator or device; and
c. a CO₂ monitoring device.

11. The system of claim 10, further comprising a fiber optic unit for viewing vocal cords.

12. The system of claim 10, wherein the jet ventilator generates jet pulses, and is capable of being adjusted for jet pulse frequency, pulse pressure (driving pressure), inspiratory/expiratory ratio (I/E) and inspiratory oxygen concentrations.

13. The system of claim 10, further comprising a mechanism for applying suction to the apparatus through the tube lumen.

14. The system of claim 10, further comprising a central control unit, wherein the central control unit comprises a sensor of breathing, a computer to integrate breathing signal and provide a triggering signal for a jet ventilator to synchronize the jet pulse from the jet ventilator with spontaneous breathing of the subject, and wherein the breathing signal is optionally provided by a breath sound detector or pressure detector located on the internal surface of said cylindrical wall.

15. The system of claim 10, wherein the CO₂ monitor measures end tidal CO₂, and the end tidal CO₂ measurement provides a triggering signal for the jet ventilator to synchronize the jet pulse from the jet ventilator with spontaneous breathing of the subject.

## Patentansprüche

1. Eine Vorrichtung für die nasale Zufuhr einer supraglottischen Jet-Ventilation, die Vorrichtung umfassend:
a. Einen länglichen flexiblen Schlauch (7) mit:
i. einer ringförmigen zylindrischen Wand, die mindestens ein Schlauchlumen definiert, das sich im Wesentlichen über dessen gesamte Länge erstreckt, diese zylindrische Wand hat eine Außenfläche (10) und eine Innenfläche (11) mit einem proximalen (9) und distalen (6) Ende,
ii. einem ersten Katheterlumen (14), das sich längs innerhalb der besagten zylindrischen Wand zwischen der Außen- und Innenfläche und entlang eines dorsalen Bereichs davon erstreckt, dieses erste Lumen hat eine erste Öffnung durch die Außenfläche der besagten zylindrischen Wand an dem proximalen Ende davon und eine zweite Öffnung durch die Innenfläche der besagten zylindrischen Wand an dessen distalem Ende,
iii. einem zweiten Katheterlumen (21), das sich längs innerhalb der besagten zylindrischen Wand entlang eines ventralen Bereichs davon erstreckt, dieses Lumen hat eine erste Öffnung durch die Außenfläche der besagten zylindrischen Wand an dem proximalen Ende davon und eine zweite Öffnung durch eine distale Seite der besagten zylindrischen Wand an dem distalen Ende der zylindrischen Wand,
b. einen ersten Katheter (4), der sich dorsal durch das erste Katheterlumen erstreckt, dieser erste Katheter hat ein proximales Ende, das sich außen an der zylindrischen Wand durch die erste Öffnung erstreckt, und ein distales Ende, das sich in den Schlauchlumen durch die zweite Öffnung erstreckt;
c. einen zweiten Katheter (2), der sich ventral durch das zweite Katheterlumen erstreckt, dieser zweite Katheter hat ein proximales Ende, das sich außen an der zylindrischen Wand durch die erste Öffnung erstreckt, und ein distales Ende, das sich in den Schlauchlumen durch die zweite Öffnung erstreckt; und
d.
i. einen Atem-Schallaufnehmer (61) und/oder Drucksensor, der sich an der Innenfläche der zylindrischen Wand befindet;
ii. eine Markierung (41) entlang der Außenfläche der zylindrischen Wand, wobei diese Markierung eine Linealmarkierung umfasst und sich an dem proximalen Teil des besagten Schlauches befindet; und wobei das Vorhandensein dieser Markierung dabei hilft, die Tiefe des nasalen Luftweges in der Nasenhöhle zu bestimmen; und
iii. einen Ring (43), der mindestens entlang des proximalen Endes des besagten länglichen flexiblen Schlauches oder eines beweglichen Stoppers verschiebbar ist.

2. Die Vorrichtung nach Anspruch 1, wobei die Vorrichtung ferner einen Atem-Schalldetektor (62) umfasst, der mit dem Atem-Schallaufnehmer (61) gekoppelt ist, und/oder einen Druckdetektor, der mit dem Drucksensor gekoppelt ist, und wobei der besagte Detektor mit einem Jet-Ventilator verbunden ist, um einen Jet Pulse einzuleiten, wenn eine Person inhaliert oder Luft einatmet.

3. Die Vorrichtung nach Anspruch 1 oder Anspruch 2, wobei die Vorrichtung ferner eine Ballonmanschette (31) umfasst und wobei diese Ballonmanschette, beim Aufblasen, dafür geeignet ist, Nasenbluten zu stoppen, indem Druck auf die Nasenwand ausgeübt wird.

4. Die Vorrichtung nach einem der Ansprüche 1-3, wobei die Vorrichtung ferner einen Befestigungsmechanismus (51) umfasst und wobei dieser Befestigungsmechanismus eine bewegliche Lasche (52) umfasst, die um einen Teil der externen Oberfläche der zylindrischen Wand gewickelt ist, und ferner einen flexiblen Draht oder ein flexibles Seil umfasst, der/das mit beiden Enden der besagten beweglichen Lasche gekoppelt und in der Lage ist, die Vorrichtung am Gesicht der Person zu befestigen, wenn die Vorrichtung auf das Gesicht der Person gesetzt wurde.

5. Die Vorrichtung nach einem der Ansprüche 1-4, wobei der erste Katheter angepasst ist, einen Jet-Ventilator oder Sauerstoffinsufflator aufzunehmen, und der zweite Katheter angepasst ist, CO₂ zu überwachen, oder wobei der erste Katheter angepasst ist, CO₂ zu überwachen und der zweite Katheter angepasst ist, einen Jet-Ventilator oder Sauerstoffinsufflator aufzunehmen.

6. Die Vorrichtung nach Anspruch 5, wobei das distale Ende entweder des ersten oder des zweiten Katheters, das angepasst ist, um den Jet-Ventilator oder den Sauerstoffinsufflator aufzunehmen, im Verhältnis zum distalen Ende des jeweils anderen des ersten oder zweiten Katheters, der angepasst ist, um CO₂ zu überwachen, in die Richtung des proximalen Endes des Schlauchlumen zurückgesetzt ist.

7. Die Vorrichtung nach einem der Ansprüche 1-6, ferner umfassend eine visuelle Überwachungseinheit für das Beobachten der Stimmbänder.

8. Die Vorrichtung nach Anspruch 7, wobei diese visuelle Überwachungseinheit in dem besagten Schlauchlumen positioniert ist.

9. Die Vorrichtung nach Anspruch 5, ferner umfassend eine Jet-Ventilations-Quelle für das Bereitstellen der Jet-Ventilation durch entweder den ersten oder den zweiten Katheter, der so angepasst ist, dass er einen Jet-Ventilator oder Sauerstoffinsufflator aufnehmen kann, wobei der CO₂- Monitor mit der Jet-Ventilations-Quelle gekoppelt ist, um einen Jet Pulse einzuleiten, wenn eine Person inhaliert oder Luft einatmet, und wobei die Jet-Ventilations-Quelle optional nach Jet Pulse-Frequenz, Impulsdruck, Einatmungs/Ausatmungs-(l/E)-Verhältnis und die Sauerstoffkonzentrationen im Jet Pulse gesteuert wird.

10. Ein System für die Ventilation und/oder Oxygenierung einer Person mit beeinträchtigter Atmung, das System umfassend:
a. Die Vorrichtung nach Anspruch 5;
b. ein(en) Jet-Ventilator oder -Gerät; und
c. ein CO₂ Überwachungsgerät.

11. Das System nach Anspruch 10, ferner umfassend eine Lichtwellenleitereinheit für das Beobachten der Stimmbänder.

12. Das System nach Anspruch 10, wobei der Jet-Ventilator Jet Pulses erzeugt und auf eine Jet Pulse-Frequenz, einen Impulsdruck (Antriebsdruck), Einatmungs/Ausatmungs-Verhältnis (l/E) und auf Sauerstoffkonzentrationen beim Einatmen eingestellt werden kann.

13. Das System nach Anspruch 10, das ferner einen Absaugmechanismus für die Vorrichtung über das Schlauchlumen umfasst.

14. Das System nach Anspruch 10, das ferner eine zentrale Steuereinheit umfasst, wobei die zentrale Steuereinheit einen Atemsensor umfasst, einen Computer, um das Atmungssignal zu integrieren und um ein Ansteuersignal für einen Jet-Ventilator bereitzustellen und den Jet Pulse aus dem Jet-Ventilator mit dem spontanen Atmen der Person zu synchronisieren, und wobei das Atmungssignal optional von einem Atem-Schalldetektor oder Druckdetektor an der Innenfläche der zylindrischen Wand bereitgestellt wird.

15. Das System nach Anspruch 10, wobei der CO₂ -Monitor das endtidale CO₂ misst und die endtidale CO₂ -Messung ein Ansteuersignal für den Jet-Ventilator bereitstellt, um den Jet Pulse aus dem Jet-Ventilator mit dem spontanen Atmen der Person zu synchronisieren.

## Revendications

1. Appareil destiné à administrer par voie nasale une ventilation par jet supraglottique, l'appareil comprenant :
a. un tube flexible de forme allongée (7) présentant :
i. une paroi cylindrique annulaire définissant au moins une lumière de tube s'étendant pratiquement sur toute la longueur de celle-ci, ladite paroi cylindrique présentant des surfaces externe (10) et interne (11) et présentant des extrémités proximale (9) et distale (6),
ii. une première lumière de cathéter (14) s'étendant sur la longueur à l'intérieur de ladite paroi cylindrique entre la surface externe et ladite surface interne et le long d'une région dorsale de celle-ci, ladite première lumière présentant une première ouverture à travers la surface externe de ladite paroi cylindrique adjacente à l'extrémité proximale de celle-ci et une seconde ouverture à travers la surface interne de ladite paroi cylindrique adjacente à l'extrémité distale de celle-ci,
iii. une seconde lumière de cathéter (21) s'étendant sur la longueur à l'intérieur de ladite paroi cylindrique le long d'une région ventrale de celle-ci, ladite lumière présentant une première ouverture à travers la surface externe de ladite paroi cylindrique adjacente à l'extrémité proximale de celle-ci et une seconde ouverture à travers une face distale de ladite paroi cylindrique à l'extrémité distale de ladite paroi cylindrique ;
b. un premier cathéter (4) s'étendant de manière dorsale à travers ladite première lumière de cathéter, ledit premier cathéter présentant une extrémité proximale s'étendant à l'extérieur de ladite paroi cylindrique à travers ladite première ouverture et présentant une extrémité distale s'étendant dans ladite lumière de tube à travers ladite seconde ouverture ;
c. un second cathéter (2) s'étendant de manière ventrale à travers ladite seconde lumière de cathéter, ledit second cathéter présentant une extrémité proximale s'étendant à l'extérieur de ladite paroi cylindrique à travers ladite première ouverture et présentant une extrémité distale s'étendant à travers ladite seconde ouverture ; et
d.
i. un capteur sonore de souffle (61) et/ou un capteur de pression situé sur la surface interne de ladite paroi cylindrique ;
ii. un marqueur (41) présent le long de la surface externe de ladite paroi cylindrique, dans lequel ledit marqueur comprend un marquage de règle et est présent sur la partie proximale dudit tube ; et dans lequel la présence dudit marqueur aide à déterminer la profondeur des voies nasales dans la cavité nasale ; et
iii. une bague (43) coulissante le long d'au moins l'extrémité proximale dudit tube flexible de forme allongée ou une butée mobile.

2. Appareil selon la revendication 1, dans lequel l'appareil comprend en outre un détecteur sonore de souffle (62) couplé audit capteur sonore de souffle (61) et/ou un détecteur de pression couplé audit capteur de pression, respectivement et dans lequel ledit détecteur est couplé à un ventilateur de jet pour déclencher une impulsion de jet lorsqu'un sujet inhale ou respire de l'air.

3. Appareil selon une quelconque de la revendication 1 ou de la revendication 2, dans lequel l'appareil comprend en outre un brassard gonflable (31) et dans lequel ledit brassard gonflable, lors du gonflage, est convenable pour arrêter le saignement nasal en exerçant une pression sur la paroi nasale.

4. Appareil selon l'une quelconque des revendications 1 à 3, dans lequel l'appareil comprend en outre en un mécanisme de fixation (51) et dans lequel ledit mécanisme de fixation comprend un clip amovible (52) enroulé autour d'une partie de la surface externe de ladite paroi cylindrique et comprend en outre un fil ou une corde souple couplé aux deux extrémités du clip amovible convenable pour immobiliser l'appareil sur le visage du sujet après le placement de l'appareil sur le visage du sujet.

5. Appareil selon l'une quelconque des revendications 1 à 4, dans lequel le premier cathéter est conçu pour accueillir un ventilateur de jet ou un insufflateur d'oxygène et le second cathéter est conçu pour surveiller le CO₂, ou dans lequel le premier cathéter est conçu pour surveiller le CO₂ et le second cathéter est conçu pour accueillir un ventilateur de jet ou un insufflateur d'oxygène.

6. Appareil selon la revendication 5, dans lequel l'extrémité distale de l'un parmi les premier et second cathéters, qui est conçue pour accueillir le ventilateur de jet ou un insufflateur d'oxygène, est en retrait par rapport à l'extrémité distale de l'autre parmi les premier et second cathéters qui est conçu pour surveiller le CO₂, dans la direction de l'extrémité proximale de la lumière de tube.

7. Appareil selon l'une quelconque des revendications 1 à 6, comprenant en outre une unité de surveillance visuelle destinée à observer les cordes vocales.

8. Appareil selon la revendication 7, dans laquelle ladite unité de surveillance visuelle est positionnée à l'intérieur de ladite lumière de tube.

9. Appareil selon la revendication 5, comprenant en outre une source de ventilation par jet destinée à la fourniture d'une ventilation par jet à travers l'un parmi les premier et second cathéters conçus pour accueillir un ventilateur de jet ou un insufflateur d'oxygène, dans lequel ledit dispositif de surveillance de CO₂ est couplé à la source de ventilation par jet pour déclencher une impulsion de jet lorsqu'un sujet inhale ou respire de l'air et dans lequel la ventilation par jet est éventuellement commandée pour la fréquence d'impulsion de jet, la pression pulsée, le ratio d'inspiration/expiration (I/E) et les concentrations d'oxygène dans l'impulsion de jet.

10. Système destiné à la ventilation et/ou l'oxygénation d'un sujet comportant une respiration altérée, le système comprenant :
a. l'appareil selon la revendication 5 ;
b. un ventilateur de jet ou un appareil ; et
c. un dispositif de surveillance du CO₂.

11. Système selon la revendication 10, comprenant en outre une unité à fibres optiques destinée à la visualisation des cordes vocales.

12. Système selon la revendication 10, dans lequel le ventilateur de jet génère des impulsions de jet et est apte à être réglé pour la fréquence d'impulsion de jet, la pression pulsée (pression de pilotage), le ratio d'inspiration/expiration (I/E) et les concentrations d'oxygène inspiratoire.

13. Système selon la revendication 10, comprenant en outre un mécanisme destiné à l'application de l'aspiration à l'appareil à travers la lumière de tube.

14. Système selon la revendication 10, comprenant en outre une unité de commande centrale, dans lequel l'unité de commande centrale comprend un capteur de respiration, un ordinateur pour intégrer le signal de respiration et fournir un signal de déclenchement destiné au ventilateur de jet pour synchroniser l'impulsion de jet provenant du ventilateur de jet avec la respiration spontanée du sujet et dans lequel le signal de respiration est fourni éventuellement par un détecteur sonore de souffle ou un détecteur de pression situé sur la surface interne de ladite paroi cylindrique.

15. Système selon la revendication 10, dans lequel le dispositif de surveillance de CO₂ mesure la fin d'expiration de CO₂, et la mesure de la fin d'expiration de CO₂ fournit un signal de déclenchement destiné au ventilateur de jet pour synchroniser l'impulsion de jet provenant du ventilateur de jet avec la respiration spontanée du sujet.
